# FASCICULE DE BREVET EUROPEEN

(11) **EP 3 046 655 B1**
(45) Date de publication et mention de la délivrance du brevet: **06.11.2019**
(21) Numéro de dépôt: 14777734.6
(22) Date de dépôt: 11.09.2014
(51) Int. Cl.: B01D 53/22

(54) **PROCÉDÉ ET INSTALLATION POUR UNE ÉPURATION FINALE DE BIOGAZ POUR PRODUIRE DU BIOMETHANE**
VERFAHREN UND VORRICHTUNG ZUR ENDREINIGUNG VON BIOGAS ZUR HERSTELLUNG VON BIOMETHAN
METHOD AND APPARATUS FOR THE FINAL PURIFICATION OF BIOGAS FOR PRODUCING BIOMETHANE

(30) Priorité: 16.09.2013 FR 1358901
(43) Date de publication de la demande: 27.07.2016
(73) Titulaire: L'Air Liquide Société Anonyme pour l'Etude et l'Exploitation des Procédés Georges Claude, 75007 Paris (FR)
(72) Inventeur: PAGET, Nicolas, 38400 Saint Martin d'Hères (FR); PRINCE, Guénaël, F-38120 Saint Egreve (FR); ZICK, Golo, F-38600 Fontaine (FR)
(74) Mandataire: Laigneau, Amandine
(86) Numéro de dépôt international: PCT/FR2014/052259
(87) Numéro de publication internationale: WO 2015/036709

(56) Documents cités:
- DE-A1-102004 007 548
- US-A1- 2004 099 138
- US-A1- 2007 125 537
- US-A1- 2013 098 242
- US-B1- 6 565 626
- US-B1- 6 630 011

## Description

La présente invention est relative à un procédé de traitement par perméation membranaire d'un courant gazeux contenant au moins du méthane et du dioxyde de carbone pour produire un courant gazeux riche en méthane - dont la teneur en méthane est conforme aux besoins de son utilisation.

Elle concerne en particulier l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs œuvrant sur un territoire (agriculteurs, industriels, pouvoirs publics), la production de biométhane aide les territoires à acquérir une plus grande autonomie énergétique.

Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié. Une première étape consiste à comprimer le biogaz qui a été produit et acheminé à pression atmosphérique, cette compression peut être obtenue - de façon classique - via un compresseur à vis lubrifiée. Les étapes suivantes visent à débarrasser le biogaz des composants corrosifs que sont le sulfure d'hydrogène et les composés organiques volatils (COV), les technologies utilisées sont de façon classique l'adsorption à pression modulée (PSA) et le piégeage sur charbon actif. Vient ensuite l'étape qui consiste à séparer le dioxyde de carbone pour disposer *in fine* de méthane à la pureté requise pour son usage ultérieur.

Le dioxyde de carbone est un contaminant typiquement présent dans le gaz naturel dont il est courant de devoir le débarrasser. Des technologies variées sont utilisées pour cela en fonction des situations ; parmi celles-ci, la technologie membranaire est particulièrement performante lorsque la teneur en CO₂ est élevée ; elle est donc particulièrement performante pour séparer le CO₂ présent dans le biogaz, et en particulier dans le gaz de décharge.

Les procédés membranaires de séparation de gaz utilisés pour la purification d'un gaz, qu'ils utilisent un ou plusieurs étages de membranes doivent permettre la production d'un gaz à la qualité requise, pour un faible coût, tout en minimisant les pertes du gaz que l'on souhaite valoriser. Ainsi, dans le cas de l'épuration du biogaz, la séparation effectuée est principalement une séparation CH₄/CO₂, devant permettre la production d'un gaz contenant en fonction de son utilisation plus de 85% de CH₄, de préférence plus de 95% de CH₄, plus préférentiellement plus de 97,5% de CH₄, tout en minimisant les pertes de CH₄ dans le gaz résiduaire et le coût d'épuration, ce dernier étant pour une part importante lié à la consommation électrique du dispositif de compression du gaz en amont des membranes.

Un des moyens classiques pour améliorer la récupération du CH₄ dans un système membranaire est d'augmenter le taux de recycle du résiduaire -opération consistant à recycler une fraction plus ou moins importante du résiduaire à l'aspiration du compresseur. La contrepartie de ce recyclage est un accroissement de la consommation électrique du compresseur, puisque que le débit à comprimer est plus important.

Afin de diminuer l'impact négatif de ce recyclage sur la consommation électrique du compresseur, une solution connue consiste à passer d'un système membranaire à un étage à un système à deux étages dans lequel le résiduaire du 1^{er} étage alimente le 2^{eme} étage, et dont uniquement le perméat du deuxième étage est recyclé, le perméat du 1^{er} étage étant quant à lui sorti du procédé (en général évacué vers l'atmosphère en traversant éventuellement un oxydateur afin d'y oxyder le méthane résiduel).

On connait aussi la solution consistant à utiliser un système membranaire à trois étages, dans lequel le perméat du 1^{er} étage subit une seconde séparation dans le troisième étage de membranes, avant d'être mélangé au perméat du 2^{eme} étage, pour être recyclé. Ce système à trois étages est utilisé sans recompression du perméat du 1^{er} étage, le perméat du 2^{eme} étage et le résiduaire du 3^{eme} étage sont recyclés à l'entrée du système membranaire. Ce système à trois étages de membranes améliore le rendement en méthane par rapport à un système à deux étages de membranes.

Cependant, si le système à trois étages de membranes permet d'améliorer notablement le taux de récupération du gaz à valoriser (le CH₄ dans le cas du biogaz) sans ajouter le coût d'un compresseur intermédiaire, il s'ensuit que sans compression intermédiaire du perméat, on n'obtient pas une bonne séparation dans le 3^{eme} étage de membrane, et ainsi, le rétentat du 3^{eme} étage de membrane contient une proportion encore importante de gaz à éliminer qui n'a pas perméé dans le 3^{eme} étage. Ce gaz à éliminer, inutilement recomprimé, augmente la consommation électrique du compresseur. Ainsi, dans le cas du biogaz, en l'absence de compression intermédiaire du perméat de la première membrane avant son passage dans le troisième étage de membrane, le rétentat du 3^{eme} étage de membrane recyclera une proportion de CO₂ à l'entrée du compresseur. Il serait donc avantageux de réduire cette proportion de CO₂ recyclé.

Le document US 2007/1255537 décrit un procédé de traitement par perméation membranaire d'un courant de gaz naturel qui utilise une installation à quatre étages de membranes plus perméables au dioxyde de carbone qu'au méthane comprenant entre autres les étapes suivantes :
- Une étape de compression dudit courant gazeux de gaz naturel dans un compresseur,
- Une étape de recyclage du quatrième retentât en aval du compresseur et
- Un recyclage du troisième perméat.

Le document US 6 565 626, quant à lui, décrit un procédé de traitement d'un flux gazeux pour éliminer simultanément l'excès d'azote et de dioxyde de carbone mettant en œuvre des membranes sélectives azote/méthane et dioxyde de carbone/méthane.

Il reste donc un besoin d'amélioration du procédé de séparation membranaire du méthane et du dioxyde de carbone contenu dans un biogaz permettant d'obtenir du méthane de grande pureté, avec un très bon rendement, tout en diminuant le coût opératoire du système.

La présente invention décrit ainsi un procédé et une installation de séparation membranaire assurant la séparation membranaire du méthane et du dioxyde de carbone contenu dans le biogaz, qui utilise judicieusement un arrangement à quatre étages de membranes ; un arrangement de quatre étages de membranes selon l'invention diminue de façon appréciable le coût de compression du gaz par rapport à l'arrangement à trois étages de membranes selon l'art antérieur tout en conservant de façon surprenante un très bon rendement en méthane. Par membrane ou étage de membranes, il convient de comprendre une membrane unique ou un faisceau de membranes ou un module composé de plusieurs faisceaux ou de plusieurs modules dès lors qu'ils sont en parallèle les uns par rapport aux autres.

L'invention propose un procédé de traitement par perméation membranaire d'un courant gazeux 1 contenant au moins du méthane et du dioxyde de carbone pour produire un courant gazeux riche en méthane comprenant au moins les étapes suivantes :
- une étape (a) de mise à disposition d'une première unité de séparation par membrane 4 munie d'une première membrane apte à recevoir un premier gaz d'alimentation et à fournir un premier perméat 6 et un premier rétentat 5, ladite première membrane étant plus perméable au dioxyde de carbone qu'au méthane,
- une étape (b) de mise à disposition d'une seconde unité de séparation par membrane 7 munie d'une seconde membrane apte à recevoir un second gaz d'alimentation et à fournir un second perméat 9 et un second rétentat 8, ladite seconde membrane étant plus perméable au dioxyde de carbone qu'au méthane, et ladite seconde unité de séparation par membrane étant connectée en série avec la première unité de séparation par membrane de telle sorte que le premier rétentat 5 constitue le second gaz d'alimentation,

- une étape (c) de mise à disposition d'une troisième unité de séparation par membrane 10 munie d'une troisième membrane apte à recevoir un troisième gaz d'alimentation et à fournir un troisième perméat 12 et un troisième rétentat 11, ladite troisième membrane étant plus perméable au dioxyde de carbone qu'au méthane, et ladite troisième unité de séparation par membrane étant connectée en série avec la première unité de séparation par membrane de telle sorte que le premier perméat 6 constitue le troisième gaz d'alimentation,
- une étape (d) de compression dudit courant gazeux contenant au moins du méthane et du dioxyde de carbone à traiter dans un compresseur 12 jusqu'à une première pression **P1**,
- une étape (e) d'alimentation de la première unité de séparation par membrane 4 avec le courant gazeux à traiter à la première pression **P1** de sorte à produire un rétentat enrichi en méthane par rapport au gaz à traiter et un perméat 6 enrichi en dioxyde de carbone par rapport au gaz à traiter,
- une étape (f) d'alimentation de la deuxième unité de séparation par membrane 7 avec le premier rétentat 5-enrichi en méthane par rapport au courant gazeux à traiter - de sorte à produire un deuxième rétentat 8 enrichi en méthane par rapport au premier rétentat 5 et un deuxième perméat 9 enrichi en dioxyde en carbone par rapport au premier rétentat 5,
- une étape (g) d'alimentation de la troisième unité de séparation par membrane 10 avec le premier perméat 6 de sorte à produire un troisième rétentat 11 enrichi en méthane par rapport au premier perméat 6, et un troisième perméat 12 enrichi en dioxyde de carbone par rapport au premier perméat 6 et mis à disposition,
- une étape (h) de recyclage du deuxième perméat au compresseur 2 de l'étape (d), caractérisé en ce que le procédé comprend en outre :
- une étape (i) de mise à disposition d'une quatrième unité de séparation par membrane 13 munie d'une quatrième membrane apte à recevoir un gaz d'alimentation et à fournir un perméat et un rétentat, ladite quatrième membrane étant plus perméable au dioxyde de carbone qu'au méthane, et ladite quatrième unité de séparation par membrane étant connectée en série avec la troisième unité de séparation par membrane de telle sorte que le troisième rétentat constitue le quatrième gaz d'alimentation,
- une étape (j) d'alimentation de la quatrième unité de séparation par membrane 13 avec le troisième rétentat 11 de sorte à produire un quatrième rétentat 14 plus riche en méthane que le troisième rétentat et un quatrième perméat 15 enrichi en dioxyde de carbone par rapport au troisième rétentat,
- une étape (k) de recyclage du quatrième rétentat au compresseur de l'étape (d),
- une étape (l) de mise à disposition ou d'évacuation du quatrième perméat 15, et
- une étape (m) d'évacuation du troisième perméat.

Les troisième et quatrième perméats sont évacués hors du procédé, ils peuvent - indépendamment ou conjointement - être par exemple traités par oxydation thermique, utilisés pour valorisation du CO₂, ou simplement rejetés à l'atmosphère.

Le procédé de séparation par membranes selon l'invention présente avantageusement tout ou partie des caractéristiques ci-dessous seules ou en combinaison :
- les membranes utilisées peuvent avoir la même sélectivité ou au moins une unité de séparation par membrane peut utiliser une membrane de sélectivité différente,
- le gaz à traiter peut être du biogaz pré-purifié, cette pré-purification pouvant selon les besoins comprendre tout ou partie des étapes suivantes : séchage, abattement du CO₂, élimination des composés organiques volatils (COV).

Selon un autre aspect de l'invention, celle-ci concerne une installation pour le traitement par perméation membranaire d'un courant gazeux 1 contenant au moins du méthane et du dioxyde de carbone pour produire un courant gazeux riche en méthane comprenant au moins:
- une première unité de séparation par membrane 4 munie d'une première membrane apte à recevoir un premier gaz d'alimentation et à fournir un premier perméat 6 et un premier rétentat 5, ladite première membrane étant plus perméable au dioxyde de carbone qu'au méthane,
- une seconde unité de séparation par membrane7 munie d'une seconde membrane apte à recevoir un second gaz d'alimentation et à fournir un second perméat 9 et un second rétentat 8, ladite seconde membrane étant plus perméable au dioxyde de carbone qu'au méthane, et ladite seconde unité de séparation par membrane étant connectée en série avec la première unité de séparation par membrane de telle sorte que le premier rétentat 5 constitue le second gaz d'alimentation,
- une troisième unité de séparation par membrane 10 munie d'une troisième membrane apte à recevoir un troisième gaz d'alimentation et à fournir un troisième perméat 12 et un troisième rétentat 11, ladite troisième membrane étant plus perméable au dioxyde de carbone qu'au méthane, et ladite troisième unité de séparation par membrane étant connectée en série avec la première unité de séparation par membrane de telle sorte que le premier perméat 6 constitue le troisième gaz d'alimentation,

- un compresseur **C2** apte à comprimer le courant gazeux contenant au moins du méthane et du dioxyde de carbone jusqu'à une première pression **P1,**
- un moyen d'alimentation de la première unité de séparation par membrane avec le courant gazeux à traiter à la première pression **P1,**
- un moyen d'alimentation de la deuxième unité de séparation par membrane avec le premier rétentat,
- un moyen d'alimentation de la troisième unité de séparation par membrane avec le premier perméat,
- un moyen de recyclage du deuxième permeat au compresseur **C2**,
caractérisé en ce que l'installation comprend en outre :
- une quatrième unité de séparation par membrane 13 plus perméable au dioxyde de carbone qu'au méthane,
- un moyen d'alimentation de la quatrième unité de séparation par membrane avec le troisième rétentat,
- un moyen de recyclage du quatrième rétentat 14 et du deuxième perméat 9 au compresseur **C2**,
- un moyen de mise à disposition ou d'évacuationdu quatrième perméat 15, et
- un moyen d'évacuation du troisième perméat 12.

L'installation comprend en outre tout ou partie des caractéristiques ci-dessous, seules ou en combinaison :
- les membranes utilisées ont la même sélectivité,
- au moins une unité de séparation par membrane utilise une membrane de sélectivité différente,
- l'installation comprend des moyens de pré-purification du biogaz à traiter aptes à assurer selon les besoins tout ou partie des fonctions de séchage, abattement du CO₂, élimination des composés organiques volatils (COV).

L'invention sera mieux comprise à l'aide de la description suivante faite en références aux figures annexées.
La figure 1 présente un schéma de principe illustrant le procédé à deux étages de membranes selon l'art antérieur.
La figure 2 présente un schéma de principe illustrant le procédé à trois étages de membranes selon l'art antérieur.
La figure 3 présente un schéma de principe illustrant le procédé à quatre étages selon l'invention.

Le tableau ci-dessous reprend les performances comparées de procédés de séparation à deux ou trois étages de membranes selon l'art antérieur avec le procédé à quatre membranes selon l'invention, ainsi qu'à titre comparatif un procédé proposant un étage de membrane supplémentaire, soit un cinquième étage de membrane en série par rapport au quatrième étage selon l'invention.

**Tableau**

| Nombre d'étages | Rendement CH₄ | Taux de recycle | Coût spécifique |
|---|---|---|---|
| 2 | 97,82 | 1 ,78 | 0,245 |
| 3 | 99,53% | 1,47 | 0,234 |
| 4 | 99,09% | 1,42 | 0,225 |
| 5 | 98,97% | 1,41 | 0,224 |

La première colonne du tableau indique le nombre de membranes utilisées

On observe ainsi que l'ajout d'un quatrième étage de membranes permet de diminuer notablement le cout spécifique.

L'ajout du quatrième étage de membrane en série sur le rétentat du 3^{eme} étage permet de diminuer encore plus la teneur en CO₂ dans le flux de recycle, et en conséquence de diminuer le débit à recomprimer. On optimise ainsi le compromis entre le coût spécifique (puissance électrique de compression) et le taux de récupération du CH₄.

On voit aussi que cet avantage est spécifique du quatrième étage, et que l'ajout d'un cinquième étage n'apporte pas d'amélioration notable au procédé, tant en terme de rendement qu'en terme de coût spécifique.

Les figures sont décrites ci-après en détail ; les éléments communs à différentes figures portent les mêmes références sur chacune des figures où ils apparaissent.

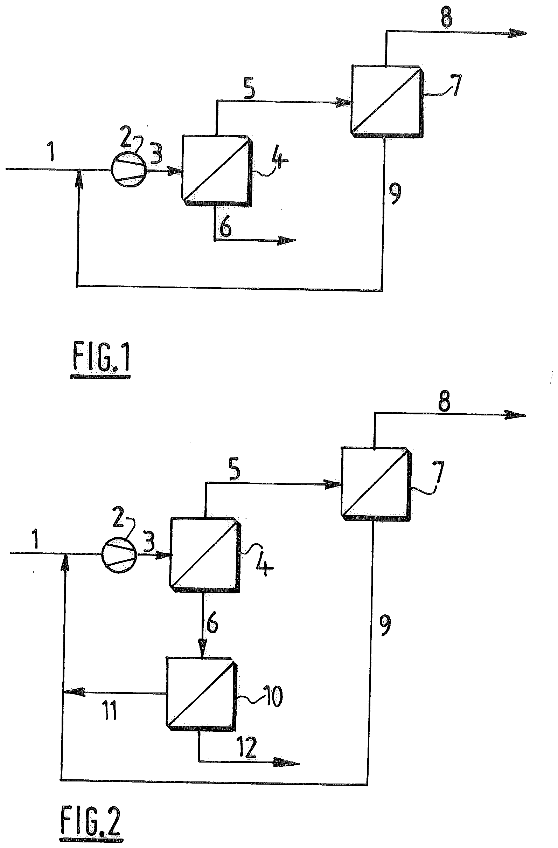

Selon le procédé connu de l'art antérieur de la figure 1 - à deux étages de membranes - le biogaz à purifier 1 est comprimé via le compresseur 2 ; le biogaz comprimé 3 à une pression P1 alimente le premier étage de membranes 4. Ce premier étage de membranes est constitué de membranes plus perméables au dioxyde de carbone qu'au méthane. En sortie de ce premier étage de membranes, on récupère un premier rétentat 5 à une pression proche de P1, et un premier perméat 6.

Le premier rétentat 5 est envoyé à l'alimentation d'un deuxième étage de membranes 7 constitué de membranes plus perméables au dioxyde de carbone qu'au méthane. En sortie de ce deuxième étage de membranes, on récupère un deuxième rétentat 8 sous pression et riche en méthane et un second perméat 9 qui est renvoyé vers le compresseur 2 pour être recomprimé avant d'être recyclé à l'alimentation du premier étage de membranes. Le premier perméat 6 se présente sous la forme d'un courant gazeux qui est sorti du procédé pour être utilisé ou rejeté.

Selon le procédé connu de l'art antérieur de la figure 2 - à trois étages de membranes - le biogaz à purifier 1 est comprimé via le compresseur 2 ; le biogaz comprimé 3 à une pression P1 alimente le premier étage de membranes référencé 4. Ce premier étage de membranes est constitué de membranes plus perméables au dioxyde de carbone qu'au méthane. En sortie de ce premier étage de membranes, on récupère un premier rétentat 5 à une pression proche de P1, et un premier perméat 6.

Le premier rétentat 5 est envoyé à l'alimentation d'un deuxième étage de membranes 7 constitué de membranes plus perméables au dioxyde de carbone qu'au méthane. En sortie de ce deuxième étage de membranes, on récupère un deuxième rétentat 8 sous pression et riche en méthane et un second perméat 9 qui est renvoyé vers le compresseur 2 pour être recomprimé avant d'être recyclé à l'alimentation du premier étage de membranes. Le premier perméat 6 se présente sous la forme d'un courant gazeux, il alimente - sans compression intermédiaire - un troisième étage de membranes référencé 10, constitué lui aussi de membranes plus perméables au dioxyde de carbone qu'au méthane. En sortie de ce troisième étage de membranes, on récupère un troisième rétentat 11 enrichi en méthane par rapport au premier perméat 6 et un troisième perméat 12. Le troisième perméat 12 est sorti du procédé pour être utilisé ou rejeté, le troisième rétentat 11 est renvoyé vers le compresseur conjointement avec le second perméat 9 pour être recomprimé avant d'alimenter le premier étage de membranes. Le troisième perméat 12 se présente sous la forme d'un courant gazeux, il est évacué du procédé pour être utilisé ou rejeté..

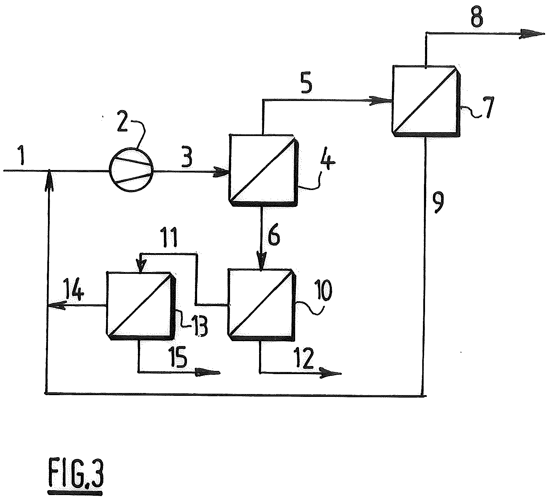

Selon le procédé selon l'invention de la figure 3, un quatrième étage de membranes est ajouté sur le rétentat du troisième étage. Le procédé de l'invention fonctionne de la manière suivante : le biogaz à purifier 1 est comprimé via le compresseur 2 ; le biogaz comprimé 3 à une pression P1 alimente le premier étage de membranes référencé 4. Ce premier étage de membranes est constitué de membranes plus perméables au dioxyde de carbone qu'au méthane. En sortie de ce premier étage de membranes, on récupère un premier rétentat 5 à une pression proche de P1, et un premier perméat 6.

Le premier rétentat 5 est envoyé à l'alimentation d'un deuxième étage de membranes 7 constitué de membranes plus perméables au dioxyde de carbone qu'au méthane. En sortie de ce deuxième étage de membranes, on récupère un deuxième rétentat 8 sous pression et riche en méthane et un second perméat 9 qui est renvoyé vers le compresseur 2 pour être recomprimé avant d'être recyclé à l'alimentation du premier étage de membranes. Le premier perméat 6 se présente sous la forme d'un courant gazeux, il alimente - sans compression intermédiaire - un troisième étage de membranes référencé 10, constitué lui aussi de membranes plus perméables au dioxyde de carbone qu'au méthane. En sortie de ce troisième étage de membranes, on récupère un troisième rétentat 11 enrichi en méthane par rapport au premier perméat 6 et un troisième perméat 12. Le troisième perméat 12 est évacué du procédé pour être utilisé ou rejeté, le troisième rétentat 11 alimente - sans compression intermédiaire - un quatrième étage de membranes référencé 13, constitué lui aussi de membranes plus perméables au dioxyde de carbone qu'au méthane. En sortie de ce quatrième étage de membranes, on récupère un quatrième rétentat 14 enrichi en méthane par rapport au troisième rétentat 11 et un quatrième perméat 15. Le quatrième perméat 15 est évacué du procédé pour être utilisé ou rejeté, le quatrième rétentat 14 est renvoyé vers le compresseur 2 conjointement avec le second perméat 9 pour être recomprimé avant d'alimenter le premier étage de membranes.

## Revendications

1. Procédé de traitement par perméation membranaire d'un courant gazeux (1) contenant au moins du méthane et du dioxyde de carbone pour produire un courant gazeux riche en méthane comprenant au moins les étapes suivantes :
une étape (a) de mise à disposition d'une première unité de séparation par membrane (4) munie d'une première membrane apte à recevoir un premier gaz d'alimentation et à fournir un premier perméat (6) et un premier rétentat (5), ladite première membrane étant plus perméable au dioxyde de carbone qu'au méthane,
une étape (b) de mise à disposition d'une seconde unité de séparation par membrane (7) munie d'une seconde membrane apte à recevoir un second gaz d'alimentation et à fournir un second perméat (9) et un second rétentat (8), ladite seconde membrane étant plus perméable au dioxyde de carbone qu'au méthane, et ladite seconde unité de séparation par membrane étant connectée en série avec la première unité de séparation par membrane de telle sorte que le premier rétentat (5) constitue le second gaz d'alimentation,
une étape (c) de mise à disposition d'une troisième unité de séparation par membrane (10) munie d'une troisième membrane apte à recevoir un troisième gaz d'alimentation et à fournir un troisième perméat (12) et un troisième rétentat (11), ladite troisième membrane étant plus perméable au dioxyde de carbone qu'au méthane, et ladite troisième unité de séparation par membrane étant connectée en série avec la première unité de séparation par membrane de telle sorte que le premier perméat (6) constitue le troisième gaz d'alimentation,
une étape (d) de compression dudit courant gazeux contenant au moins du méthane et du dioxyde de carbone à traiter dans un compresseur (2) jusqu'à une première pression **P1**,
une étape (e) d'alimentation de la première unité de séparation par membrane (4) avec le courant gazeux à traiter à la première pression **P1** de sorte à produire un rétentat (5) enrichi en méthane par rapport au gaz à traiter et un perméat (6) enrichi en dioxyde de carbone par rapport au gaz à traiter,
une étape (f) d'alimentation de la deuxième unité de séparation par membrane (7) avec le premier rétentat (5)-enrichi en méthane par rapport au courant gazeux à traiter - de sorte à produire un deuxième rétentat (8) enrichi en méthane par rapport au premier rétentat (5) et un deuxième perméat (9) enrichi en dioxyde en carbone par rapport au premier rétentat (5),
une étape (g) d'alimentation de la troisième unité de séparation par membrane (10) avec le premier perméat (6) de sorte à produire un troisième rétentat (11) enrichi en méthane par rapport au premier perméat (6), et un troisième perméat (12) enrichi en dioxyde de carbone par rapport au premier perméat (6),
une étape (h) de recyclage du deuxième perméat au compresseur (2) de l'étape (d), **caractérisé en ce que** le procédé comprend en outre :
une étape (i) de mise à disposition d'une quatrième unité de séparation par membrane (13) munie d'une quatrième membrane apte à recevoir un gaz d'alimentation et à fournir un perméat et un rétentat, ladite quatrième membrane étant plus perméable au dioxyde de carbone qu'au méthane, et ladite quatrième unité de séparation par membrane étant connectée en série avec la troisième unité de séparation par membrane de telle sorte que le troisième rétentat constitue le quatrième gaz d'alimentation,
une étape (j) d'alimentation de la quatrième unité de séparation par membrane (13) avec le troisième rétentat (11) de sorte à produire un quatrième rétentat (14) plus riche en méthane que le troisième rétentat et un quatrième perméat (15) enrichi en dioxyde de carbone par rapport au troisième rétentat,
une étape (k) de recyclage du quatrième rétentat conjointement avec le deuxième perméat au compresseur de l'étape (d),
une étape (l) de mise à disposition ou d'évacuation du quatrième perméat (15), et
une étape (m) d'évacuation du troisième perméat.

2. Procédé selon la revendication 1 dans lequel les membranes utilisées ont la même sélectivité.

3. Procédé selon la revendication 1 dans lequel au moins une unité de séparation par membrane utilise une membrane de sélectivité différente.

4. Procédé selon l'une des revendications précédentes dans lequel le gaz à traiter est du biogaz pré-purifié,

5. Installation pour le traitement par perméation membranaire d'un courant gazeux (2) contenant au moins du méthane et du dioxyde de carbone pour produire un courant gazeux riche en méthane comprenant au moins:
une première unité de séparation par membrane (4) munie d'une première membrane apte à recevoir un premier gaz d'alimentation et à fournir un premier perméat (6) et un premier rétentat (5), ladite première membrane étant plus perméable au dioxyde de carbone qu'au méthane,
une seconde unité de séparation par membrane (7) munie d'une seconde membrane apte à recevoir un second gaz d'alimentation et à fournir un second perméat (9) et un second rétentat (8), ladite seconde membrane étant plus perméable au dioxyde de carbone qu'au méthane, et ladite seconde unité de séparation par membrane étant connectée en série avec la première unité de séparation par membrane de telle sorte que le premier rétentat (5) constitue le second gaz d'alimentation,
une troisième unité de séparation par membrane (10) munie d'une troisième membrane apte à recevoir un troisième gaz d'alimentation et à fournir un troisième perméat (12) et un troisième rétentat (11), ladite troisième membrane étant plus perméable au dioxyde de carbone qu'au méthane, et ladite troisième unité de séparation par membrane étant connectée en série avec la première unité de séparation par membrane de telle sorte que le premier perméat (6) constitue le troisième gaz d'alimentation.
un compresseur **C(2)** apte à comprimer le courant gazeux contenant au moins du méthane et du dioxyde de carbone jusqu'à une première pression **P1**,
un moyen d'alimentation de la première unité de séparation par membrane avec le courant gazeux à traiter à la première pression **P1,**
un moyen d'alimentation de la deuxième unité de séparation par membrane avec le premier rétentat,
un moyen d'alimentation de la troisième unité de séparation par membrane avec le premier perméat,
un moyen de recyclage du deuxième perméat au compresseur **C (2)**,
**caractérisé en ce que** l'installation comprend en outre :
une quatrième unité de séparation par membrane (13) plus perméable au dioxyde de carbone qu'au méthane,
un moyen d'alimentation de la quatrième unité de séparation par membrane avec le troisième rétentat,
un moyen de recyclage conjoint du quatrième rétentat (14) et du deuxième perméat (19) au compresseur **C(2)**,
un moyen de mise à disposition ou d'évacuation du quatrième perméat (15), et
un moyen d'évacuation du troisième perméat (12).

6. Installation selon la revendication 5 dans laquelle les membranes utilisées ont la même sélectivité.

7. Installation selon la revendication 5 dans laquelle au moins une unité de séparation par membrane utilise une membrane de sélectivité différente.

8. Installation selon l'une quelconque des revendications 5 à 7 comprenant en outre des moyens de pré-purification du biogaz à traiter aptes à assurer selon les besoins tout ou partie des fonctions de séchage, abattement du CO₂, élimination des composés organiques volatils (COV).

## Patentansprüche

1. Verfahren zur Behandlung durch Membranpermeation eines gasförmigen Stroms (1), der mindestens Methan und Kohlendioxid enthält, um einen methanreichen gasförmigen Strom herzustellen, mindestens die folgenden Schritte umfassend:
einen Schritt (a) der Bereitstellung einer ersten Membranzerlegungseinheit (4), die mit einer ersten Membran ausgestattet ist, die in der Lage ist, ein erstes Zuführungsgas aufzunehmen, und ein erstes Permeat (6) und ein erstes Retentat (5) bereitzustellen, wobei die erste Membran für Kohlendioxid permeabler ist als für Methan,
einen Schritt (b) der Bereitstellung einer zweiten Membranzerlegungseinheit (7), die mit einer zweiten Membran ausgestattet ist, die in der Lage ist, ein zweites Zuführungsgas aufzunehmen und ein zweites Permeat (9) und ein zweites Retentat (8) bereitzustellen, wobei die zweite Membran für Kohlendioxid permeabler ist als für Methan, und wobei die zweite Membranzerlegungseinheit mit der ersten Membranzerlegungseinheit seriell verbunden ist, sodass das erste Retentat (5) das zweite Zuführungsgas bildet,
einen Schritt (c) der Bereitstellung einer dritten Membranzerlegungseinheit (10), die mit einer dritten Membran ausgestattet ist, die in der Lage ist, ein drittes Zuführungsgas aufzunehmen und ein drittes Permeat (12) und ein drittes Retentat (11) bereitzustellen, wobei die dritte Membran für Kohlendioxid permeabler ist als für Methan, und die dritte Membranzerlegungseinheit mit der ersten Membranzerlegungseinheit seriell verbunden ist, sodass das erste Permeat (6) das dritte Zuführungsgas bildet,
einen Schritt (d) der Verdichtung des gasförmigen Stroms, der mindestens Methan und Kohlendioxid enthält, das in einem Kompressor (2) behandelt werden soll, auf einen ersten Druck **P1,**
einen Schritt (e) der Versorgung der ersten Membranzerlegungseinheit (4) mit dem zu behandelnden gasförmigen Strom bei dem ersten Druck **P1**, um ein mit Methan angereichertes Retentat (5) in Bezug auf das zu behandelnde Gas und ein mit Kohlendioxid angereichertes Permeat (6) in Bezug auf das zu behandelnde Gas herzustellen,
einen Schritt (f) der Versorgung der zweiten Membranzerlegungseinheit (7) mit dem ersten Retentat (5), das in Bezug auf den zu behandelnden gasförmigen Strom mit Methan angereichert ist, um ein zweites Retentat (8), das in Bezug auf das erste Retentat (5) mit Methan angereichert ist, und ein zweites Permeat (9), das in Bezug auf das erste Retentat (5) mit Kohlendioxid angereichert ist, herzustellen,
einen Schritt (g) der Versorgung der dritten Membranzerlegungseinheit (10) mit dem ersten Permeat (6), um ein drittes Retentat (11), das in Bezug auf das erste Permeat (6) mit Methan angereichert ist, und ein drittes Permeat (12), das in Bezug auf das erste Permeat (6) mit Kohlendioxid angereichert ist, herzustellen,
einen Schritt (h) des Recyclings des zweiten Permeats (2) zu dem Kompressor von Schritt (d), **dadurch gekennzeichnet, dass** das Verfahren weiter umfasst:
einen Schritt (i) der Bereitstellung einer vierten Membranzerlegungseinheit (13), die mit einer vierten Membran ausgestattet ist, die in der Lage ist, ein Zuführungsgas aufzunehmen und ein Permeat und ein Retentat bereitzustellen, wobei die vierte Membran für Kohlendioxid permeabler ist als für Methan, und wobei die vierte Membranzerlegungseinheit mit der dritten Membranzerlegungseinheit seriell verbunden ist, sodass das dritte Retentat das vierte Zuführungsgas bildet,
einen Schritt (j) der Versorgung der vierten Membranzerlegungseinheit (13) mit dem dritten Retentat (11), um ein viertes Retentat (14) herzustellen, das reicher an Methan ist als das dritte Retentat und ein viertes Permeat (15), das in Bezug auf das dritte Retentat mit Kohlendioxid angereichert ist,
einen Schritt (k) des Recyclings des vierten Retentats zusammen mit dem zweiten Permeat zum Kompressor von Schritt (d),
einen Schritt (l) der Bereitstellung oder Evakuierung des vierten Permeats (15), und
einen Schritt (m) der Evakuierung des dritten Permeats.

2. Verfahren nach Anspruch 1, wobei die verwendeten Membranen die gleiche Selektivität aufweisen.

3. Verfahren nach Anspruch 1, wobei mindestens eine Membranzerlegungseinheit eine Membran mit unterschiedlicher Selektivität verwendet.

4. Verfahren nach einem der vorstehenden Ansprüche, wobei das zu behandelnde Gas vorgereinigtes Biogas ist,

5. Installation zur Behandlung durch Membranpermeation eines gasförmigen Stroms (2), der mindestens Methan und Kohlendioxid enthält, um einen methanreichen gasförmigen Strom herzustellen, der mindestens umfasst:
eine erste Membranzerlegungseinheit (4), die mit einer ersten Membran ausgestattet ist, die in der Lage ist, ein erstes Zuführungsgas aufzunehmen, und ein erstes Permeat (6) und ein erstes Retentat (5) bereitzustellen, wobei die erste Membran für Kohlendioxid permeabler ist als für Methan,
eine zweite Membranzerlegungseinheit (7), die mit einer zweiten Membran ausgestattet ist, die in der Lage ist, ein zweites Zuführungsgas aufzunehmen und ein zweites Permeat (9) und ein zweites Retentat (8) bereitzustellen, wobei die zweite Membran für Kohlendioxid permeabler ist als für Methan, und wobei die zweite Membranzerlegungseinheit mit der ersten Membranzerlegungseinheit seriell verbunden ist, sodass das erste Retentat (5) das zweite Zuführungsgas bildet,
eine dritte Membranzerlegungseinheit (10), die mit einer dritten Membran ausgestattet ist, die in der Lage ist, ein drittes Zuführungsgas aufzunehmen und ein drittes Permeat (12) und ein drittes Retentat (11) bereitzustellen, wobei die dritte Membran für Kohlendioxid permeabler ist als für Methan, und die dritte Membranzerlegungseinheit mit der ersten Membranzerlegungseinheit seriell verbunden ist, sodass das erste Permeat (6) das dritte Zuführungsgas bildet.
einen Kompressor **C(2),** der in der Lage ist, den gasförmigen Strom, der mindestens Methan und Kohlendioxid enthält, auf einen ersten Druck **P1** zu verdichten,
ein Mittel der Versorgung der ersten Membranzerlegungseinheit mit dem zu behandelnden gasförmigen Strom bei dem ersten Druck **P1,**
ein Mittel der Versorgung der zweiten Membranzerlegungseinheit mit dem ersten Retentat,
ein Mittel der Versorgung der dritten Membranzerlegungseinheit mit dem ersten Permeat,
ein Mittel des Recyclings des zweiten Permeats zum Kompressor **C(2),**
**dadurch gekennzeichnet, dass** die Vorrichtung weiter umfasst:
eine vierte Membranzerlegungseinheit (13), die für Kohlendioxid permeabler ist als für Methan,
ein Mittel der Versorgung der vierten Membranzerlegungseinheit mit dem dritten Retentat,
ein Mittel des gemeinsamen Recyclings des vierten Retentats (14) und des zweiten Permeats (19) zum Kompressor **C(2)**,
ein Mittel der Bereitstellung oder Evakuierung des vierten Permeats (15), und
ein Evakuierungsmittel des dritten Permeats (12).

6. Installation nach Anspruch 5, wobei die verwendeten Membranen die gleiche Selektivität aufweisen.

7. Installation nach Anspruch 5, wobei mindestens eine Membranzerlegungseinheit eine Membran mit unterschiedlicher Selektivität verwendet.

8. Installation nach einem der Ansprüche 5 bis 7, weiter umfassend Mittel der Vorreinigung des zu behandelnden Biogases, die in der Lage sind, je nach Bedarf alle oder einen Teil der Funktionen der Trocknung, der Senkung von CO₂, der Eliminierung flüchtiger organischer Verbindungen (VOC) durchführen.

## Claims

1. Method for treating, by membrane permeation, a gas stream (1) containing at least methane and carbon dioxide to produce a methane-rich gas stream comprising at least the following steps:
a step (a) of making available a first membrane separation unit (4) provided with a first membrane capable of receiving a first supply gas and of providing a first permeate (6) and a first retentate (5), said first membrane being more permeable to carbon dioxide than to methane,
a step (b) of making available a second membrane separation unit (7) provided with a second membrane capable of receiving a second supply gas and of providing a second permeate (9) and a second retentate (8), said second membrane being more permeable to carbon dioxide than to methane, and said second membrane separation unit being connected in series with the first membrane separation unit, such that the first retentate (5) constitutes the second supply gas,
a step (c) of making available a third membrane separation unit (10) provided with a third membrane capable of receiving a third supply gas and of providing a third permeate (12) and a third retentate (11), said third membrane being more permeable to carbon dioxide than to methane, and said third membrane separation unit being connected in series with the first membrane separation unit, such that the first permeate (6) constitutes the third supply gas,
a step (d) of compressing said gas stream containing at least methane and carbon dioxide to be treated in a compressor (2) up to a first pressure P1,
a step (e) of providing the first membrane separation unit (4) with the gas stream to be treated at the first position P1 so as to produce a methane-rich retentate (5) with respect to the gas to be treated and a carbon dioxide-rich permeate (6) with respect to the gas to be treated,
a step (f) of providing the second membrane separation unit (7) with the first methane-rich retentate (5) with respect to the gas stream to be treated - so as to produce a second methane-rich retentate (8) with respect to the first retentate (5) and a second carbon dioxide-rich permeate (9) with respect to the first retentate (5),
a step (g) of providing a third membrane separation unit (10) with the first permeate (6) so as to produce a third methane-rich retentate (11) with respect to the first permeate (6), and a third carbon dioxide-rich permeate (12) with respect to the first permeate (6),
a step (h) of recycling the second permeate in the compressor (2) of step (d), **characterised in that** the method further comprises:
a step (i) of making available a fourth membrane separation unit (13) provided with a fourth membrane capable of receiving a supply gas and of providing a permeate and a retentate, said fourth membrane being more permeable to carbon dioxide than to methane, and said fourth membrane separation unit being connected in series with the third membrane separation unit, such that the third retentate constitutes the fourth supply gas,
a step (j) of providing the fourth membrane separation unit (13) with the third retentate (11) so as to produce a fourth retentate (14) richer in methane than the third retentate and a fourth carbon dioxide-rich permeate (15) with respect to the third retentate,
a step (k) of recycling the fourth retentate jointly with the second permeate in the compressor of step (d),
a step (l) of making available or evacuating the fourth permeate (15), and
a step (m) of evacuating the third permeate.

2. Method according to claim 1, wherein the membranes used have the same selectivity.

3. Method according to claim 1, wherein at least one membrane separation unit uses a membrane of different selectivity.

4. Method according to one of the preceding claims, wherein the gas to be treated is pre-purified biogas.

5. Installation for the treatment by membrane permeation of a gas stream (2) containing at least methane and carbon dioxide to produce a methane-rich gas stream comprising at least:
a first membrane separation unit (4) provided with a first membrane capable of receiving a first supply gas and of providing a first permeate (6) and a first retentate (5), said first membrane being more permeable to carbon dioxide than or methane,
a second membrane separation unit (7) provided with a second membrane, capable of receiving a second supply gas and of providing a second permeate (9) and a second retentate (8), said second membrane being more permeable to carbon dioxide than to methane, and said second membrane separation unit being connected in series with the first membrane separation unit, such that the first retentate (5) constitutes the second supply gas,
a third membrane separation unit (10) provided with a third membrane capable of receiving a third supply gas and of providing a third permeate (12) and a third retentate (11), said third membrane being more permeable to carbon dioxide than to methane, and said third membrane separation unit being connected in series with the first membrane separation unit, such that the first permeate (6) constitutes the third supply gas,
a compressor C(2) capable of compressing the gas stream containing at least methane and carbon dioxide up to a first pressure P1,
a means for providing the first membrane separation unit with the gas stream to be treated to the first pressure P1,
a means for providing the second membrane separation unit with the first retentate,
a means for providing the third membrane separation unit with the first permeate,
a means for recycling the second permeate in the compressor C(2),
**characterised in that** the installation further comprises:
a fourth membrane separation unit (13) more permeable to carbon dioxide than to methane,
a means for providing the fourth membrane separation unit with the third retentate,
a means for jointly recycling the fourth retentate (14) and the second permeate (19) in the compressor C(2),
a means for making available or evacuating the fourth permeate (15), and
a means for evacuating the third permeate (12).

6. Installation according to claim 5, wherein the membranes used have the same selectivity.

7. Installation according to claim 5, wherein at least one membrane separation unit uses a membrane of different selectivity.

8. Installation according to any one of claims 5 to 7, further comprising means for pre-purifying biogas to be treated, capable of ensuring, depending on need, all or some of the drying functions, CO₂ allowance, removal of volatile organic compounds (VOCs).
